# EUROPEAN PATENT APPLICATION

(11) **EP 3 005 934 A1**
(43) Date of publication of application: **13.04.2016**
(21) Application number: 14803760.9
(22) Date of filing: 08.05.2014
(51) Int. Cl.: A61B 1/04, A61B 1/00

(54) **MEDICAL DEVICE**

(30) Priority: 31.05.2013 JP 2013115773
(71) Applicant: OLYMPUS CORPORATION, Shibuya-ku Tokyo 151-0072 (JP)
(72) Inventor: TAKAHASHI, Kazuhiko, Tokyo 151-0072 (JP)
(74) Representative: Gunzelmann, Rainer
(86) International application number: PCT/JP2014/062341
(87) International publication number: WO 2014/192512

(57) **Abstract**

A medical device is provided which performs a display in which a user can easily and intuitively know a position in a subject where an image is captured. A medical device 1 includes a feature information calculation unit 141 that calculates feature information representing a feature of each image, an image classification unit 143 that classifies a series of images into a plurality of groups in time sequence according to the similarity between the images, a position axis average color bar generation unit 145 that generates a position axis average color bar in which pieces of the feature information are arranged in time sequence in a specified area on a screen, a display area calculation unit 144 that calculates a display area for each group, where feature information of images belonging to each of the plurality of groups is displayed, in the position axis average color bar, based on the number of groups of the plurality of groups, and a display unit 15 that displays the position axis average color bar. The position axis average color bar generation unit 145 arranges the feature information of images belonging to each of the plurality of groups in the display area for each group calculated by the display area calculation unit 144.

## Description

### Field

The present invention relates to a medical device for displaying an image acquired by a medical image acquiring apparatus such as a capsule endoscope.

### Background

In recent years, in the field of endoscope, an examination using a capsule endoscope which is inserted into a subject such as a patient and captures an image inside the subject is known. The capsule endoscope is an apparatus in which an imaging function, a wireless communication function, and the like are included in a capsule-shaped casing formed into a size that can be introduced into a digestive tract of the subject. The capsule endoscope sequentially and wirelessly transmits image data generated by capturing an image inside the subject to the outside of the subject. A series of image data wirelessly transmitted from the capsule endoscope is temporarily accumulated in a receiving device provided outside the subject and transferred (downloaded) from the receiving device to an image processing device such as a workstation. In the image processing device, various image processing operations are applied to the image data and thereby a series of images of an organ or the like inside the subject are generated.

Here, the time required for one examination using the capsule endoscope is about eight hours and the number of images acquired in the examination amounts to about 60,000. Therefore, it takes a very long time to observe all the images. Further, among these images, there may be a plurality of redundant scenes obtained by repeatedly capturing images of the same region while the capsule endoscope stays at the same region, and it is inefficient to observe all of such scenes along a time series. Therefore, a summarizing technique that summarizes images in which scene changes are small is proposed in order to efficiently observe a series of images in a short time (for example, see Patent Literature 1).

Meanwhile, in the examination using the capsule endoscope, the capsule endoscope is moved by a peristaltic movement of the subject, so that a user such as a doctor cannot control the position of the endoscope. Therefore, it is difficult for the user to know what portion in the subject or what portion in an organ such as small intestine is imaged by only observing an image acquired by the capsule endoscope.

Therefore, a technique is developed which displays a bar that indicates the entire imaging period of images captured by the capsule endoscope (for example, see Patent Literature 2). The bar is generated by detecting average color of each image from color information of image data and arranging the average colors in a belt-shaped area in order from the earliest captured image to the latest captured image. The bar is also called an average color bar. In Patent Literature 2, a movable slider is displayed on the average color bar and an image corresponding to the position of the slider is displayed on a screen. Here, an image capturing order of the images (the number of captured images) corresponds to an image capturing time (the elapsed time from the start of image capturing), so that a user can know an approximate image capturing time of an image currently being observed by referring to the average color bar.

### Citation List

### Patent Literature

Patent Literature 1: Japanese Patent Application Laid-open No. 2009-160298
Patent Literature 2: Japanese Patent Application Laid-open No. 2004-337596

### Summary

### Technical Problem

However, since the capsule endoscope does not move at a constant speed at all times in the subject, time information indicated by the average color bar does not necessarily correspond to position information of the capsule endoscope. Therefore, even when a user refers to the average color bar, it is difficult for the user to intuitively know the image capturing position in the subject in an image currently being observed.

The present invention has been made in view of the foregoing and an object of the invention is to provide a medical device which performs a display in such a way that a user can easily and intuitively know the image capturing position in the subject.

### Solution to Problem

To solve the problem described above and to achieve the object, a medical device according to the invention is a medical device for displaying a series of images acquired in time sequence on a screen. The medical device includes: a feature information calculation unit configured to calculate feature information representing a feature of each image included in the series of images; a classification unit configured to classify the series of images into a plurality of groups in time sequence according to similarity between the images; a feature information display generation unit configured to generate a feature information display in which pieces of the feature information are arranged in time sequence in a specified area on the screen; a display area calculation unit configured to calculate a display area for each group, where feature information of images belonging to each of the plurality of groups is displayed, in a display area of the feature information display, based on the number of the plurality of groups; and a display unit configured to display the feature information display. The feature information display generation unit is configured to arrange the feature information of images belonging to each of the plurality of groups, in time sequence, on the display area for each group calculated by the display area calculation unit.

In the above-described medical device, the display area calculation unit is configured to calculate the display area for each group by equally dividing the display area of the feature information display by the number of the plurality of groups.

In the above-described medical device, the display area calculation unit is configured to calculate a display area for each image, where the feature information of each image belonging to the group is displayed, in the display area for each group, based on the number of images belonging to the group.

In the above-described medical device, the display area calculation unit is configured to calculate the display area for each image by equally dividing the display area for each group by the number of images belonging to the group, and the feature information display generation unit is configured to arrange the feature information of each image belonging to the group, in time sequence, on the display area for each image calculated by the display area calculation unit.

In the above-described medical device, the feature information display generation unit is configured to arrange feature information of a representative image of images belonging to each of the plurality of groups on the display area for each group.

In the above-described medical device, the feature information display generation unit is configured to arrange a representative value of the feature information of images belonging to each of the plurality of groups on the display area for each group.

In the above-described medical device, the classification unit is configured to classify the series of images into a plurality of groups in time sequence according to variation between the images.

In the above-described medical device, the series of images is a series of in-vivo images acquired by a capsule endoscope that is configured to be inserted into a subject to capture images while moving inside the subject. Advantageous Effects of Invention

According to the invention, the display area for each group in the feature information display is calculated based on the number of the plurality of groups in time sequence classified according to the similarity between images, and the feature information of images belonging to the group is arranged on the display area for each group, so that the feature information display can be a display that roughly corresponds to the scene changes in images, that is, the change in the image capturing position. Therefore, a user can easily and intuitively know the image capturing position in the subject by referring to the feature information display as described above.

### Brief Description of Drawings

FIG. 1 is a block diagram illustrating a configuration example of a medical device according to an embodiment of the present invention.
FIG. 2 is a schematic diagram illustrating an example of a screen displayed on a display unit in FIG. 1.
FIG. 3 is a schematic diagram illustrating a series of in-vivo images acquired along a time series.
FIG. 4 is a schematic diagram for explaining a generation method of a time axis average color bar.
FIG. 5 is a schematic diagram illustrating in-vivo images classified into a plurality of groups in time sequence order.
FIG. 6 is a schematic diagram for explaining a generation method of a position axis average color bar.
FIG. 7 is a schematic diagram for explaining the generation method of the position axis average color bar.
FIG. 8 is a schematic diagram illustrating a configuration example of a capsule endoscope system to which the medical device illustrated in FIG. 1 is applied. Description of Embodiment

Hereinafter, a medical device according to an embodiment of the present invention will be described with reference to the drawings. The present invention is not limited by the embodiment. The same reference signs are used to designate the same elements throughout the drawings

### (Embodiment)

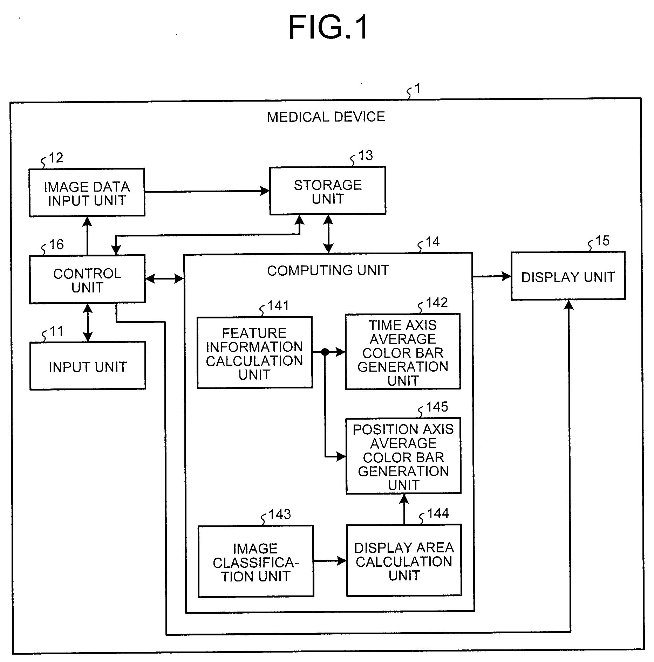

FIG. 1 is a block diagram illustrating a configuration example of a medical device according to an embodiment of the present invention. As illustrated in FIG. 1, the medical device 1 according to the embodiment is formed by a general-purpose computer such as, for example, a workstation and a personal computer, and includes an input unit 11, an image data input unit 12, a storage unit 13, a computing unit 14, a display unit 15, and a control unit 16.

The input unit 11 includes input devices such as a keyboard, various buttons, and various switches, and pointing devices such as a mouse and a touch panel, and inputs a signal according to an operation of a user to these devices into the control unit 16.

The image data input unit 12 is an interface that can connect with a USB or a communication line such as a wired LAN and a wireless LAN. The image data input unit 12 includes a USB port, a LAN port, and the like. The image data input unit 12 receives image data and related information through an external device connected to the USB port and various lines and stores the image data and the related information in the storage unit 13.

The storage unit 13 includes a semiconductor memory such as a flash memory, a RAM, and a ROM, a recording medium such as an HDD, an MO, a CD-R, and a DVD-R, a driving device that drives the recording medium and the like. The storage unit 13 stores a program and various information for causing the medical device 1 to operate and perform various functions, image data inputted into the image data input unit 12 and the like.

The computing unit 14 includes hardware such as, for example, a CPU. The computing unit 14 generates display image data by applying image processing such as white balance processing, demosaicing, color conversion, density conversion (gamma conversion or the like), smoothing (noise removal or the like), and sharpening (edge enhancement or the like) to the image data stored in the storage unit 13 and performs processing for generating an observation screen of a specified format including an image by reading the program stored in the storage unit 13. The detailed configuration and operation of the computing unit 14 will be described later.

The display unit 15 is a display device such as a CRT display or a liquid crystal display. The display unit 15 displays an observation screen of a specified format including an image, and other information under the control of the control unit 15.

The control unit 16 includes hardware such as, for example, a CPU, and transfers instructions and data to each unit included in the medical device 1 based on a signal and the like inputted from the input unit 11 to integrally control the operation of the entire medical device 1 by reading the program stored in the storage unit 13.

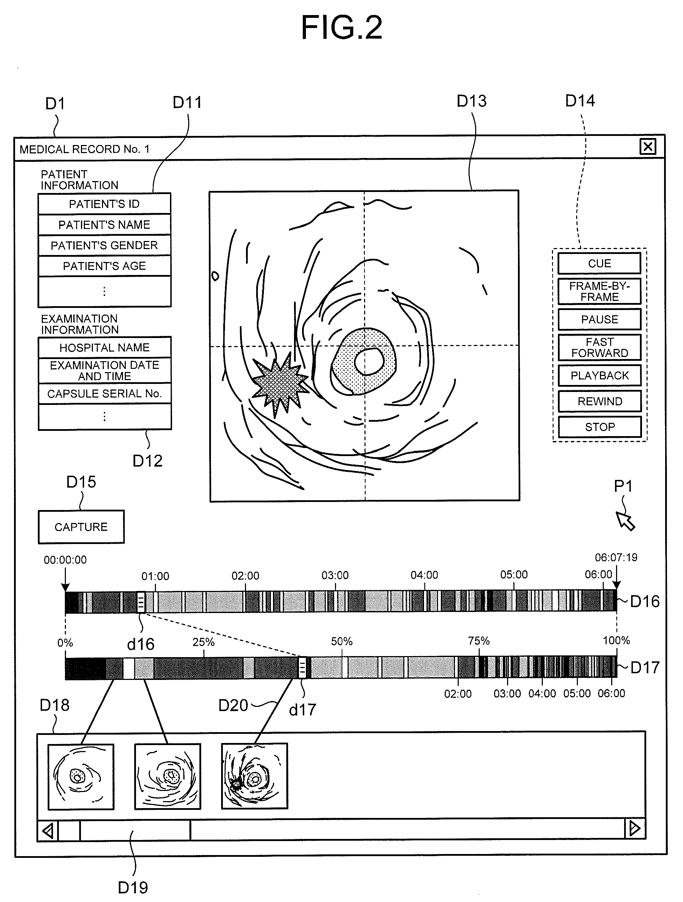

FIG. 2 is a schematic diagram illustrating an example of the observation screen displayed on the display unit 15. The observation screen is a screen that displays a series of in-vivo images acquired by a capsule endoscope that is inserted into a subject and captures images at a given cycle (for example, two frames/second) while moving inside the subject.

As illustrated in FIG. 2, the observation screen D1 includes patient information D11 for identifying a patient who is a subject, examination information D12 for identifying an examination performed on the patient, a main display area D13 in which a series of in-vivo images acquired by the examination are played back, a playback operation button group D14 that is used when controlling the playback of the in-vivo images in the main display area D13, a capture button D15 that is used when capturing an in-vivo image that is currently being displayed in the main display area D13, a time axis average color bar D16 that is a belt-shaped image in which pieces of feature information of the series of in-vivo images are arranged along an image capturing time axis, a position axis average color bar D17 that is a belt-shaped image in which pieces of feature information of the series of in-vivo images are arranged in time sequence order according to scene changes of the images, and a captured image display area D18 in which captured in-vivo images or reduced images are displayed as thumbnails in a list.

The playback operation button group D14 is a set of buttons used when a user inputs an instruction to control the playback of the in-vivo images in the main display area D13. The playback operation button group D14 includes, for example, a cue button, a frame-by-frame button, a pause button, a fast forward button, a playback button, a rewind button, and a stop button.

The capture button D15 is a button used by the user to input an instruction to capture the in-vivo image that is currently being displayed in the main display area D13. When the capture button D15 is clicked by an operation of a pointer P1 on the observation screen D1 using the input unit 11, a flag for identifying image data as a captured image is added to image data corresponding to the in-vivo image displayed in the main display area D13 at that time. Thereby, the captured image is registered.

The time axis average color bar D16 is a feature information display in which average colors, which are pieces of feature information of the in-vivo images, are arranged along the image capturing time axis in a belt-shaped area. The user can check how the average color (in particular, tendency of red) of the in-vivo images varies through a series of in-vivo images by referring to the time axis average color bar D16.

The time axis average color bar D16 is provided with a slider d16 that indicates a position on the time axis average color bar D16 corresponding to the in-vivo image that is currently being displayed in the main display area D13. While the in-vivo images are automatically being played back in the main display area D13, the slider d16 moves on the time axis average color bar D16 according to the image capturing time of the in-vivo image that is currently being displayed. In the present application, the image capturing time is the time elapsed from the start of image capturing (the start of the examination). The user can know the time when the in-vivo image that is currently being displayed is captured by referring to the slider d16. Further, the user can display an in-vivo image of the image capturing time corresponding to the position of the slider d16 in the main display area D13 by moving the slider d16 along the time axis average color bar D16 by the operation of the pointer P1 on the observation screen D1 using the input unit 11.

The position axis average color bar D17 is a feature information display in which average colors, which are pieces of feature information of the in-vivo images, are arranged in time sequence in a belt-shaped area, and the position axis average color bar D17 is an area in which the length (the length of the longitudinal direction of the belt-shaped area) of a display area of an average color is changed for each in-vivo image according to scene changes of the in-vivo images. More specifically, the length of a display area of an average color is set to short for in-vivo images which are acquired by repeatedly capturing images of the same portion in the subject because the capsule endoscope stays at the same region and where scene changes are small. On the other hand, the length of a display area of an average color is set to long for in-vivo images where there are many scene changes because the moving speed of the capsule endoscope is high. In this way, the horizontal axis of the position axis average color bar D17 is associated with the frequency of scene changes of the in-vivo images. The scene changes of the in-vivo images are mainly generated by the change of the image capturing position of the capsule endoscope, so that it can be assumed that the length of a display area of an average color of each in-vivo image corresponds to the speed of change of the image capturing position, that is, the moving speed of the capsule endoscope. Therefore, it can be said that the horizontal axis of the position axis average color bar D17 artificially represents the image capturing position of an in-vivo image in the subject.

In FIG. 2, the image capturing position of an in-vivo image in the subject is represented by percentage where an introduction position (mouth) of the capsule endoscope into the subject corresponding to the image capturing start time (00:00:00) is 0% and an excretion position (anus) of the capsule endoscope corresponding to the image capturing end time (06:07:19) is 100%. The user can check how the average color (in particular, tendency of red) of the in-vivo images varies according to the position in the subject by referring to the position axis average color bar D17.

The position axis average color bar D17 is provided with a slider d17 that indicates a position on the position axis average color bar D17 corresponding to the in-vivo image that is currently being displayed in the main display area D13. In the same manner as the slider d16, while the in-vivo images are automatically being played back in the main display area D13, the slider d17 moves on the position axis average color bar D17 according to the in-vivo image that is currently being displayed. The user can roughly know the position where the in-vivo image that is currently being displayed is captured in the subject by referring to the slider d17. Further, the user can display an in-vivo image at a desired position in the subject in the main display area D13 by moving the slider d17 along the position axis average color bar D17 by the operation of the pointer P1 on the observation screen D1 using the input unit 11.

The slider d16 and the slider d17 are linked with each other, so that when one slider is moved by an operation of the user, the other slider is also moved by the linkage with the one slider.

The captured image display area D18 is an area in which in-vivo images registered as the captured images, or reduced images (hereinafter referred to as thumbnail images) of these images are displayed in time sequence in a list. The captured image display area D18 is provided with a slider D19 used to slide a display range. A connecting line D20 may be provided which indicates a correspondence relationship between a thumbnail image in the captured image display area D18 and a position on the time axis average color bar D16 or the position axis average color bar D17.

Next, a detailed configuration of the computing unit 14 will be described. As illustrated in FIG. 1, the computing unit 14 includes a feature information calculation unit 141 that calculates feature information representing a feature of each image, a time axis average color bar generation unit 142 that generates the time axis average color bar D16, an image classification unit 143 that classifies a series of images into a plurality of groups in time sequence according to the similarity between the images, a display area calculation unit 144 that calculates a display area of the feature information of each image in the position axis average color bar D17, and a position axis average color bar generation unit 145 that generates the position axis average color bar D17.

The feature information calculation unit 141 calculates the feature information of each image by applying specified image processing such as average color calculation processing, red color detection processing, lesion detection processing, and organ detection processing to the image data stored in the storage unit 13. In the embodiment, as an example, an average color calculated by the average color calculation processing is used as the feature information.

The time axis average color bar generation unit 142 generates the time axis average color bar D16 by arranging the feature information of each image calculated by the feature information calculation unit 141 at a position corresponding to the image capturing time of the image in the display area of the time axis average color bar D16 illustrated in FIG. 2.

The image classification unit 143 classifies a series of images corresponding to the image data stored in the storage unit 13 into a plurality of groups in time sequence according to the similarity between the images.

The display area calculation unit 144 calculates a display area of the feature information of each image arranged in the position axis average color bar D17 illustrated in FIG. 2 based on a classification result by the image classification unit 143.

The position axis average color bar generation unit 145 is a feature information display generation means that generates the position axis average color bar D17 by arranging the feature information of each image at the display area calculated by the display area calculation unit 144.

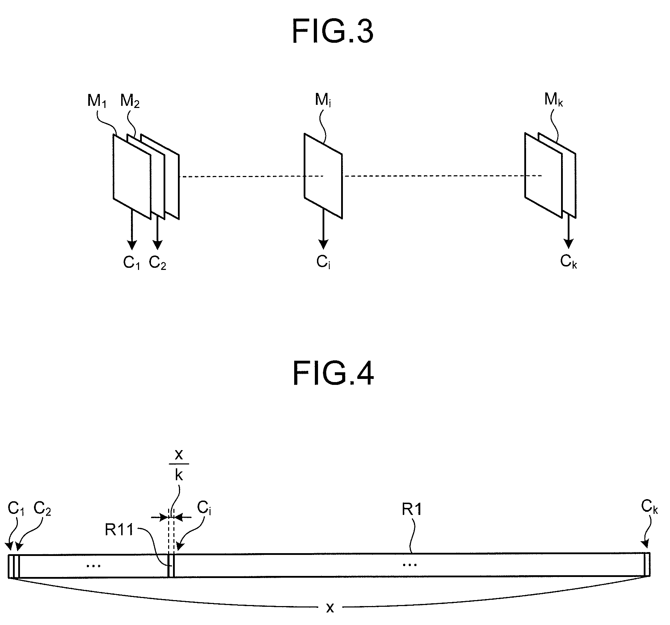

Next, an operation of the computing unit 14 will be described with reference to FIGS. 3 to 7. In the description below, as an example, processing for a series of in-vivo images acquired by a capsule endoscope that captures an image at a given cycle T (frames/second) will be described.

When the image data acquired in time sequence by the capsule endoscope is inputted into the image data input unit 12 and stored in the storage unit 13, the computing unit 14 generates display image data by applying specified image processing to the image data. As illustrated in FIG. 3, the feature information calculation unit 141 calculates an average value (average color Cᵢ) of pixel values of pixels included in each in-vivo image Mᵢ (i = 1 to k) as the feature information for a series of in-vivo images M₁ to Mₖ (k is the total number of images) acquired in time sequence.

Subsequently, as illustrated in FIG. 4, the time axis average color bar generation unit 142 calculates a display area R11 for each image in a display area R1 in the time axis average color bar D16 (see FIG. 2). Specifically when the number of pixels in the longitudinal direction of the display area R1 is x, a display area having a uniform length (x/k pixels) is assigned to each in-vivo image Mᵢ by dividing the number of pixels x by the number of the in-vivo images Mᵢ.

Further, the time axis average color bar generation unit 142 arranges the average colors Cᵢ of the in-vivo images Mᵢ, each of which has x/k pixels, in time sequence from an end (for example, the left end) of the display area R1. In other words, the average color Cᵢ of the ith in-vivo image Mᵢ is arranged at the ((x/k)×i)th pixel from the end of the time axis average color bar D16. In this way, the time axis average color bar D16 in which the display position of the average color Cᵢ of the in-vivo image Mᵢ corresponds to the image capturing time (i/T) is generated.

When processing is performed on the in-vivo images acquired by the capsule endoscope, the number k of the in-vivo images (for example, 60,000 images) is greater than the number x of pixels in the display area R1 (for example, 1,000 pixels). In this case, average colors of a plurality of in-vivo images Mᵢ are assigned to one pixel, so that an average value of the average colors Cᵢ of the plurality of in-vivo images Mᵢ is actually displayed at the one pixel.

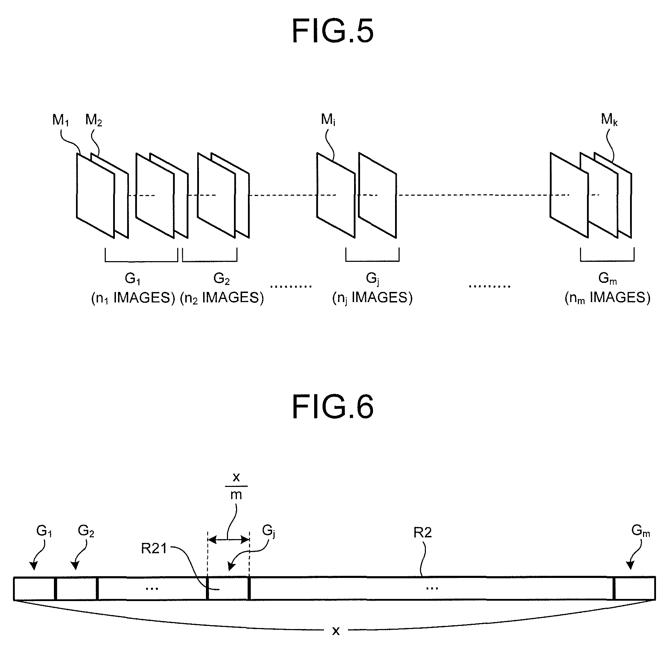

On the other hand, as illustrated in FIG. 5, the image classification unit 143 classifies a series of in-vivo images M₁ to Mₖ into a plurality of groups G₁ to Gₘ (m is the total number of groups) in time sequence according to the similarity between the images. When classifying the in-vivo images M₁ to Mₖ, a known image summarization technique can be used. In the description below, as an example, an image summarization technique disclosed in Japanese Patent Application Laid-open No. 2009-160298 will be described.

First, at least one feature area is extracted from each image of a series of images to be summarized. As a result, the image is divided into a plurality of partial areas. Further, the entire image may be extracted as the feature area. In this case, the image may not be divided. Subsequently, variation between an image and a comparison image (for example, an adjacent image in a time series) is calculated for each partial area. Then, the variation between the images for each partial area is compared with a threshold value that is set for each partial area based on feature data of the feature area, and values of comparison results are accumulated, so that a total variation of each image is calculated. The total variation is compared with a specified threshold value, so that a scene change image (a summarized image) where scene changes between the images are large is extracted.

The image classification unit 143 sequentially extracts the scene change images from the series of in-vivo images M₁ to Mₖ by using such an image summarization technique, and classifies in-vivo images from a certain scene change image to an in-vivo image Mᵢ immediately before the next scene change image as one group Gⱼ (j = 1 to m). Then, image data of each in-vivo image Mᵢ is added with information such as an order j in time sequence of a group Gⱼ to which the in-vivo image Mᵢ belongs, the number nⱼ of in-vivo images that belong to the group Gⱼ, and an order in time sequence of the in-vivo image Mᵢ in the group Gⱼ, and the image data is stored in the storage unit 13.

According to the image classification method as described above, the number of scene changes of the in-vivo images Mᵢ is small in a section in which the capsule endoscope stays or moves slowly, so that the number of extracted scene change images is small. As a result, many in-vivo images belong to one group Gⱼ. On the other hand, the number of scene changes of the in-vivo images Mᵢ is large in a section in which the capsule endoscope moves fast, so that the number of extracted scene change images is large. As a result, the number nⱼ of in-vivo images that belong to one group Gⱼ is small.

Subsequently, as illustrated in FIG. 6, the display area calculation unit 144 calculates a display area R21 for each group in a display area R2 of the position axis average color bar D17 based on the number of groups m into which the in-vivo images Mᵢ are classified. Specifically, when the number of pixels in the longitudinal direction of the display area R2 is x, a display area having a uniform length (x/m pixels) is assigned to each group Gⱼ by dividing the number of pixels x by the number of groups m.

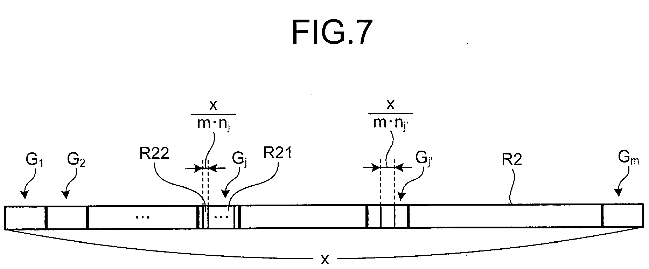

Subsequently, as illustrated in FIG. 7, the display area calculation unit 144 calculates a display area R22 for each image based on the number nⱼ of in-vivo images that belong to each group Gⱼ. Specifically, the display area R21 of the group Gⱼ including x/m pixels is divided by the number nⱼ of in-vivo images, and a display area having a uniform length (x/(m×nⱼ) pixels) is assigned to each in-vivo image Mᵢ belonging to the group Gⱼ. In this way, the length of the display area R22 for each image is proportional to the reciprocal of the number nⱼ of in-vivo images that belong to the same group Gⱼ. Therefore, for example, as in the case of group Gⱼ and group G_{j'}, when the numbers of in-vivo images belonging to the groups are different from each other (nⱼ # n_{j'}), the length of the display area R22 for each image varies for each group (x/(m×nⱼ) ≠ x/ (m×n_{j'})).

The position axis average color bar generation unit 145 arranges the average colors Cᵢ of the in-vivo images Mᵢ, each of which has x/(m×nⱼ) pixels, in time sequence from an end (for example, the left end) of the display area R2. In the same manner as in the time axis average color bar D16, when average colors of a plurality of in-vivo images Mᵢ are assigned to one pixel, an average value of the average colors Cᵢ of the in-vivo images Mᵢ is displayed at the one pixel.

In such a position axis average color bar D17, a ratio of the display area R22 for each image to the entire display area R2 (1/(m×nⱼ)) varies depending on a degree of scene changes, that is, the degree of change of the image capturing position (the position of the capsule endoscope). For example, when the change of the image capturing position is small, the number nⱼ of in-vivo images that belong to one group is relatively large, so that the aforementioned ratio is small. On the other hand, when the change of the image capturing position is large, the number nⱼ of in-vivo images that belong to one group is relatively small, so that the aforementioned ratio is large. Therefore, by arranging the average color Cᵢ of the in-vivo image Mᵢ on the display area R22 for each image, the display position of the average color Cᵢ of each in-vivo image Mᵢ is artificially associated with the image capturing position in the subject.

As described above, according to the embodiment, the display area of the average color Cᵢ of each in-vivo image Mᵢ is calculated by using a result of classifying a series of in-vivo images M₁ to Mₖ according to the similarity, so that the position axis average color bar D17 associated with the scene changes of the in-vivo images Mᵢ, that is, the change of the image capturing position, can be generated. Therefore, a user can easily and intuitively know the image capturing position of the in-vivo image Mᵢ in the subject by referring to the position axis average color bar D17.

Further, according to the embodiment, the image capturing position of the in-vivo image Mᵢ that is currently being displayed is indicated by percentage on the position axis average color bar D17, so that the user can more intuitively know the image capturing position of the in-vivo image Mᵢ that is currently being displayed.

In the above description, the average color is calculated as the feature information of the in-vivo image Mᵢ. However, in addition to the average color, information obtained by the red color detection processing, the lesion detection processing, the organ detection processing, and the like may be used as the feature information. For example, when red color information obtained by the red color detection processing is used as the feature information, the user can easily and intuitively know a position of a lesion such as bleeding.

In the above description, the feature information of each in-vivo image Mᵢ is arranged in the position axis average color bar D17. However, the feature information for each group may be arranged on the display area R21 for each group. Specifically, feature information of a representative image (for example, an in-vivo image detected as a scene change image) of in-vivo images belonging to each group Gⱼ is arranged on the display area R21 for each group having x/m pixels. Alternatively, a representative value (for example, an average value or a mode value) of the feature information of in-vivo images belonging to each group Gⱼ may be arranged. In this case, it is possible to simplify arithmetic processing for generating the position axis average color bar D17. Further, the user can roughly know how the feature information (for example, average color) in the subject varies according to a position in the subject.

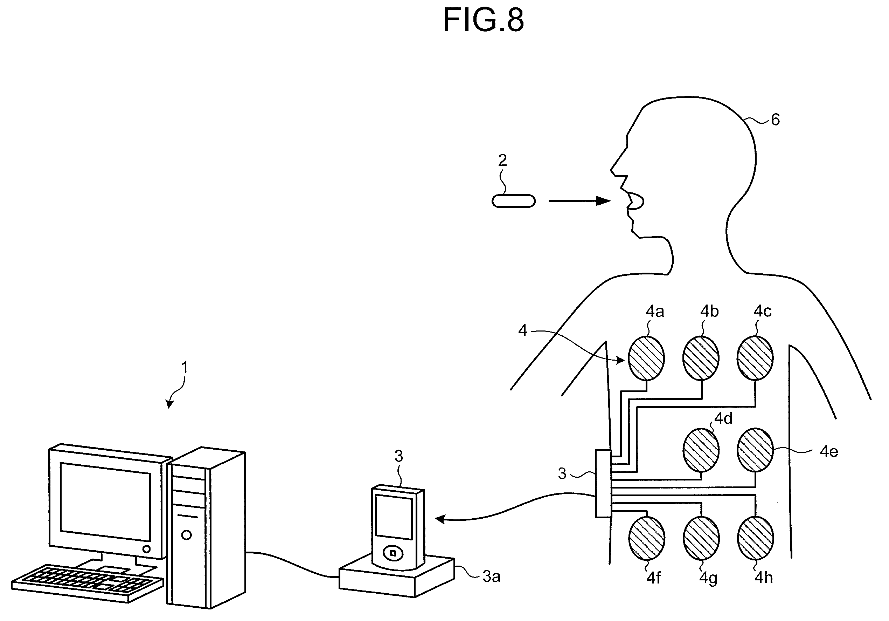

FIG. 8 is a schematic diagram illustrating a system configuration example in which the medical device according to the above embodiment is applied to a capsule endoscope system. The capsule endoscope system illustrated in FIG. 8 includes a capsule endoscope 2 which is inserted into a subject 6 and which generates image data by capturing images in the subject 6 and wirelessly transmits the image data, and a receiving device 3 that receives the image data transmitted from the capsule endoscope 2 through a receiving antenna unit 4 attached to the subject 6, in addition to the medical device 1.

The capsule endoscope 2 is an apparatus in which a capsule-shaped casing having a size that can be swallowed by the subject 6 includes an imaging element such as a CCD, an illumination element such as an LED, a memory, a wireless communication means, and other various components. The capsule endoscope 2 generates image data by applying signal processing to an imaging signal outputted from the imaging element and transmits the image data and related information (serial number of the capsule endoscope 2 and the like) by superimposing the image data and the related information on a wireless signal.

The receiving device 3 includes a signal processing unit that demodulates the wireless signal transmitted from the capsule endoscope 2 and performs specified signal processing on the demodulated wireless signal, a memory that stores image data, a data transmitting unit that transmits the image data to an external device, and the like. The data transmitting unit is an interface that can connect with a USB or a communication line such as a wired LAN and a wireless LAN.

The receiving device 3 receives the wireless signal transmitted from the capsule endoscope 2 through the receiving antenna unit 4 including a plurality of (in FIG. 8, eight) receiving antennas 4a to 4h and acquires the image data and the related information by demodulating the wireless signal. The receiving antennas 4a to 4h are formed by using, for example, loop antennas, and are arranged at specified positions on an outer surface of the subject 6 (for example, at positions corresponding to organs in the subject 6, which are a passage of the capsule endoscope 2).

In the capsule endoscope system illustrated in FIG. 8, when the receiving device 3 is set on a cradle 3a connected to a USB port of the medical device 1, the receiving device 3 is connected with the medical device 1. When the receiving device 3 is connected with the medical device 1, the image data accumulated in the memory of the receiving device 3 is transferred to the medical device 1 and the processing for the series of in-vivo images M₁ to Mₖ described above is performed.

The present invention described above is not limited to the embodiment, but may be variously modified according to specifications and the like. For example, the present invention may be formed by removing some components from all the components described in the above embodiment. From the above description, it is obvious that other various embodiments can be made within the scope of the present invention.

### Reference Signs List

- 1: Medical device

- 2: Capsule endoscope
- 3: Receiving device
- 3a: Cradle
- 4: Receiving antenna unit
- 4a to 4h: Receiving antenna
- 6: Subject
- 11: Input unit
- 12: Image data input unit
- 13: Storage unit
- 14: Computing unit
- 15: Display unit
- 16: Control unit
- 141: Feature information calculation unit
- 142: Time axis average color bar generation unit
- 143: Image classification unit
- 144: Display area calculation unit
- 145: Position axis average color bar generation unit
- D1: Observation screen
- D11: Patient information
- D12: Examination information
- D13: Main display area
- D14: Playback operation button group
- D15: Capture button
- d16, d17, D19: Slider
- D16: Time axis average color bar
- D17: Position axis average color bar
- D18: Captured image display area
- D20: Connecting line
- P1: Pointer
- R1, R2: Display area
- R11, R22: Display area for each image
- R21: Display area for each group

## Claims

1. A medical device for displaying a series of images acquired in time sequence on a screen, the medical device comprising:
a feature information calculation unit configured to calculate feature information representing a feature of each image included in the series of images;
a classification unit configured to classify the series of images into a plurality of groups in time sequence according to similarity between the images;
a feature information display generation unit configured to generate a feature information display in which pieces of the feature information are arranged in time sequence in a specified area on the screen;
a display area calculation unit configured to calculate a display area for each group, where feature information of images belonging to each of the plurality of groups is displayed, in a display area of the feature information display, based on the number of the plurality of groups; and
a display unit configured to display the feature information display,
wherein the feature information display generation unit is configured to arrange the feature information of images belonging to each of the plurality of groups, in time sequence, on the display area for each group calculated by the display area calculation unit.

2. The medical device according to claim 1, wherein the display area calculation unit is configured to calculate the display area for each group by equally dividing the display area of the feature information display by the number of the plurality of groups.

3. The medical device according to claim 1, wherein the display area calculation unit is configured to calculate a display area for each image, where the feature information of each image belonging to the group is displayed, in the display area for each group, based on the number of images belonging to the group.

4. The medical device according to claim 3, wherein
the display area calculation unit is configured to calculate the display area for each image by equally dividing the display area for each group by the number of images belonging to the group, and
the feature information display generation unit is configured to arrange the feature information of each image belonging to the group, in time sequence, on the display area for each image calculated by the display area calculation unit.

5. The medical device according to claim 1, wherein the feature information display generation unit is configured to arrange feature information of a representative image of images belonging to each of the plurality of groups on the display area for each group.

6. The medical device according to claim 1, wherein the feature information display generation unit is configured to arrange a representative value of the feature information of images belonging to each of the plurality of groups on the display area for each group.

7. The medical device according to claim 1, wherein the classification unit is configured to classify the series of images into a plurality of groups in time sequence according to variation between the images.

8. The medical device according to claim 1, wherein the series of images is a series of in-vivo images acquired by a capsule endoscope that is configured to be inserted into a subject to capture images while moving inside the subject.
